(19) Europäisches Patentamt
European Patent Office
Office européen des brevets

(11) EP 2 426 099 A1

(12) # EUROPEAN PATENT APPLICATION

(43) Date of publication:
**07.03.2012 Bulletin 2012/10**

(21) Application number: **11175610.2**

(22) Date of filing: **27.07.2011**

(51) Int Cl.:
***C07C 41/09*** *(2006.01)* ***C07C 43/10*** *(2006.01)*
***C09D 5/02*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **08.06.2011 US 494586 P**
**30.08.2010 US 402465 P**

(71) Applicants:
- **Rohm and Haas Company**
**Philadelphia,**
**Pennsylvania 19106-2399 (US)**
- **Dow Global Technologies LLC**
**Midland, MI 48674 (US)**

(72) Inventors:
- **Van Dyk, Antony Keith**
**Blue Bell, Pennsylvania 19422 (US)**
- **Tulchinsky, Michael L.**
**Midland, Michigan 48642 (US)**

(74) Representative: **Buckley, Guy Julian**
**Patent Outsourcing Limited**
**1 King Street**
**Bakewell**
**Derbyshire DE45 1DZ (GB)**

(54) **Coalescent for aqueous compositions**

(57) A coalescent composition selected from 1,3-($C_6$-$C_{12}$alkyloxy)-2-propanol, 1,1'-oxybis[3-($C_6$-$C_{12}$alkyloxy)]-2-propanol, and mixtures thereof is provided. Preferred coalescents are 1,3-(decyloxy)-2-propanol and 1,1'-oxybis[3-(heptyloxy)]-2-propanol. A method for forming glycerol diethers and diglycerol diethers, an aqueous coating composition including the coalescent compositions and a method for forming a coating are also provided.



Printed by Jouve, 75001 PARIS (FR)

## Description

This invention relates to a coalescent for aqueous compositions. This invention particularly relates to a coalescent composition selected from the group consisting of: 1,3-($C_6$-$C_{12}$alkyloxy)-2-propanol, 1,1'-oxybis[3-($C_6$-$C_{12}$alkyloxy)]-2-propanol, and mixtures and isomers thereof. The invention also relates to a method for forming the coalescent composition, an aqueous coating composition including an emulsion polymer and from 0.1% to 40% by weight, based on the weight of emulsion polymer solids, of the coalescent composition and a method for forming a dry coating.

Compliance with the increasingly stringent Volatile Organic Compound (VOC) regulations around the world is a major challenge for the aqueous formulation chemists today. In most coating formulations the use of an organic solvent or an external plasticizer is required to facilitate film formation. One of the major contributors to the VOC as well as to film integrity and the coating properties dependent thereon in a coating formulation is the coalescent in the formulation. Desirably, coalescents are not volatile organic compounds (VOC), preferably are based on renewable resources, are hydrolytically stable, non-yellowing, have no odor, are nontoxic, non-hazardous, non-flammable, and facilitate high performance in the dry coatings. While having very low vapor pressure, the compounds should exhibit a large diffusion coefficient (high mobility in an emulsion polymer matrix). While partitioning primarily to the emulsion polymer phase, the coalescents should also have appreciable water solubility to allow efficient transport to the emulsion polymer during the paint making process, but remain partitioned to the emulsion polymer, and not migrate.

The present invention serves to provide coalescent compositions that are particularly suitable for use in aqueous compositions such as aqueous decorative and protective coatings for various substrates which coatings provide a sought-after balance of coatings properties, particularly including desirable application properties such as, for example, flow and leveling, and facile film formation and hardness/block resistance development while maintaining desirable dry coatings properties, particularly wherein the aqueous compositions contain low VOC and advantageously low toxicity.

U.S. Patent Application Publication No. 20100048940 discloses new polyol ether compounds, and processes for their preparation that may be used as solvents, degreasers, wetting agents, emulsifying agents, lubricants and intermediates for surfactants. The compounds are disclosed to be suitable for use in cleaning compositions, coatings, perfumery and inks. Improvements in the VOC/coatings properties profile of previously disclosed coalescents are still desired. It has been found that the coalescent compositions of the present invention fill that need.

In a first aspect of the present invention there is provided a coalescent composition selected from the group consisting of: 1,3-($C_6$-$C_{12}$alkyloxy)-2-propanol, 1,1'-oxybis[3-($C_6$-$C_{12}$alkyloxy)]-2-propanol, and mixtures and isomers thereof.

In a second aspect of the present invention there is provided a method for forming glycerol diethers or diglycerol diethers comprising: 1) subjecting an aldehyde and a molar excess of glycerol or diglycerol to reductive etherification conditions to form a glycerol monoether or a diglycerol monoether; and 2) subjecting an aldehyde and a molar excess of said glycerol monoether or said diglycerol monoether to reductive etherification conditions to form said glycerol diethers or said diglycerol diethers.

In a third aspect of the present invention there is provided an aqueous coating composition comprising an emulsion polymer and from 0.1% to 40% by weight, based on the weight of emulsion polymer solids, said coalescent composition of the first aspect of the present invention.

In a fourth aspect of the present invention there is provided a method for forming a coating comprising (a) forming the aqueous coating composition of the second aspect of the present invention; (b) applying said aqueous coating composition to a substrate; and (c) drying, or allowing to dry, said applied aqueous coating composition.

The coalescent composition of the present invention includes compounds selected from the group consisting of: 1,3-($C_6$-$C_{12}$alkyloxy)-2-propanol, 1,1'-oxybis[3-($C_6$-$C_{12}$alkyloxy)]-2-propanol, and mixtures and isomers thereof. "Alkyl" herein includes all carbon/hydrogen groups such as for example, linear alkyl, branched alkyl, alicyclic, and aromatic groups. Preferred coalescent compositions are selected from: 1,3-($C_8$-$C_{12}$alkyloxy)-2-propanol, 1,1'-oxybis[3-($C_6$-$C_{10}$alkyloxy)]-2-propanol, and mixtures and isomers thereof. More preferred coalescent compositions are 1,3-(decyloxy)-2-propanol and 1,1'-oxybis[3-(heptyloxy)]-2-propanol. By "coalescent composition" is meant a composition that facilitates the film formation of an aqueous polymeric composition, particularly an aqueous coating composition that includes a dispersion of polymer in an aqueous medium such as, for example, a polymer prepared by emulsion polymerization techniques.

The coalescent composition of the present invention may be formed by known methods such catalytic reductive etherification. However, the preparation of dialkyl ethers of glycerol by catalytic reductive etherification by methods disclosed in the art gave poor selectivity and low yields. US Patent No. 5,446,210 discloses catalytic reductive etherification of aldehydes or ketones with glycerol using 5%Pd/C at high hydrogen pressure (1,500 psi) that gives low selectivity to dialkyl ethers of glycerol. For example, formation of glycerol bis(isopropyl) ethers was only 10% by weight. US Patent No. 6,011,071 also disclosed low selectivity to the glycerol diethers under similar conditions.

In one aspect of the present invention an efficient preparation of glycerol diethers and diglycerol diethers is

conducted in a two step reductive etherification process starting from the corresponding aldehydes and glycerol or diglycerol. The first step involves the reductive etherification of an aldehyde with an excess of glycerol or diglycerol to give a glycerol monoether or diglycerol monoether. The second step comprises the reductive etherification of the aldehyde with an excess of the intermediate glycerol monoether or diglycerol monoether to give the desired glycerol diether or diglycerol diether. The product in the first reaction serves as a starting material in the second reaction, with or without purification or isolation of the monoether.

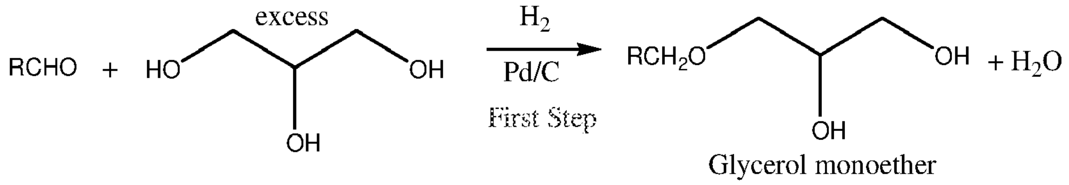

Glycerol monoether

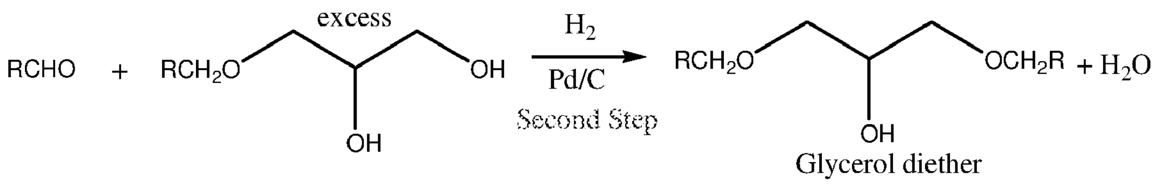

Glycerol diether

According to the prior art, this transformation gives a low selectivity to the diether if conducted in a one step fashion using a 2:1 excess of the aldehyde or ketone over glycerol. US 5,446,210 discloses the catalytic reductive etherification of aldehydes or ketones with glycerol using 5%Pd/C at 1,500 psi of hydrogen that gives low selectivity to dialkyl ethers of glycerol. For example, the formation of glycerol bis(isopropyl) ethers from acetone and glycerol (2:1 molar ratio) was only 10% of the reaction mixture by weight.

The coalescent composition of the present invention is ideally supplied in the form of a low viscosity, readily pourable, fluid at room temperature. Ideally the coalescent composition remains liquid even at out-door temperatures which can be 0 °C and lower. Various additives, including impurities, can also be beneficial in promoting the dispersibility of coalescent compounds into coating compositions. To this end it is sometimes desirable to retain a controllable amount of impurities such as un-reacted or partially reacted components and byproducts in the coalescent composition. Highly purified materials are known to have higher melting points than materials with some impurities. While the method of this invention is capable of producing highly purified compounds, it is advantageously also capable of producing less highly purified material.

The aqueous coating composition of the present invention includes an emulsion polymer and from 0.1% to 40% by weight, based on the weight of emulsion polymer solids, of the coalescent composition of the present invention. In one embodiment when the MFFT of the emulsion polymer is from -20°C to 60°C, from 0.1% to 30% coalescent composition, by weight based on the weight of emulsion polymer solids, may be used. Alternatively, when the MFFT of the emulsion polymer is from -20°C to 30°C, from 0.1% to 5% coalescent composition, by weight based on the weight of emulsion polymer solids, may be used.

The minimum film formation temperature ("MFFT") of the emulsion polymer is typically from -5°C to 100 °C, alternatively from -5 °C to 60 °C or from -5°C to 30°C. See, for example, A. Toussaint, M. De Wilde, F. Molenaar, J. Mulvihill "Calculation of Tg and MFFT depression due to added coalescing agents" Progress in Organic Coatings 30 (1997) 179-184. MFFTs of the emulsion polymers herein are those measured using an SS-3000 Gardener MFFT bar.

The emulsion polymer typically includes at least one copolymerized ethylenically unsaturated monomer such as, for example, styrene or substituted styrenes; vinyl toluene; butadiene; (meth)acrylonitrile; a (meth)acrylic ester monomer such as, for example, methyl (meth)acrylate, ethyl (meth)acrylate, butyl (meth)acrylate, hydroxyethyl (meth)acrylate, hydroxypropyl (meth)acrylate, and ureido-functional (meth)acrylates; vinyl acetate or other vinyl esters; vinyl monomers such as vinyl chloride, vinylidene chloride, and N-vinyl pyrollidone. The use of the term "(meth)" followed by another term such as (meth)acrylate, as used throughout the disclosure, refers to both acrylates and methacrylates.

In certain embodiments the emulsion polymer includes from 0% to 6%, or in the alternative, from 0% to 3 wt% or from 0% to 1%, by weight based on the weight of the polymer, of a copolymerized multi-ethylenically unsaturated monomer. It is important to select the level of multi-ethylenically unsaturated monomer so as to not materially interfere with film formation and integrity. Multi-ethylenically unsaturated monomers include, for example, allyl (meth)acrylate, diallyl phthalate, 1,4-butylene glycol di(meth)acrylate, 1,2-ethylene glycol di(meth)acrylate, 1,6-hexanediol di(meth)acrylate, and divinyl benzene.

The emulsion polymer includes from 0% to 15%, preferably from 0.5% to 5%, of a copolymerized monoethylenically-unsaturated acid monomer, based on the weight of the polymer. Acid monomers include carboxylic acid monomers such as, for example, (meth)acrylic acid, crotonic acid, itaconic acid, fumaric acid, maleic acid, monomethyl itaconate, monomethyl fumarate, monobutyl fumarate, maleic anhydride, 2-acrylamido-2-methylpropane sulfonic acid, vinyl sulfonic acid, styrene sulfonic acid, 1-allyloxy-2-hydroxypropane sulfonic acid, alkyl allyl sulfosuccinic acid, sulfoethyl (meth)acrylate, phosphoalkyl (meth)acrylates such as phosphoethyl (meth)acrylate, phosphopropyl (meth)acrylate, and phosphobutyl (meth)acrylate, phosphoalkyl crotonates, phosphoalkyl maleates, phosphoalkyl fumarates, phosphodialkyl (meth)acrylates, phosphodialkyl crotonates, and allyl phosphate.

The aqueous emulsion polymer is typically formed by an addition polymerization emulsion polymerization process as is known in the art. Conventional surfactants and blends may be used including, for example, anionic and/or nonionic emulsifiers such as, for example, alkali metal or ammonium alkyl sulfates, alkyl sulfonic acids, fatty acids, and oxyethylated alkyl phenols, and mixtures thereof. Polymerizable surfactants that include at least one ethylenically unsaturated carbon-carbon bond which can undergo free radical addition polymerization may be used. The amount of surfactant used is usually 0.1% to 6% by weight, based on the weight of total monomer. Either thermal or redox initiation processes may be used. Conventional free radical initiators may be used such as, for example, hydrogen peroxide, t-butyl hydroperoxide, t-amyl hydroperoxide, ammonium and/or alkali persulfates, typically at a level of 0.01% to 3.0% by weight, based on the weight of total monomer. Redox systems using the same initiators coupled with a suitable reductant such as, for example, sodium sulfoxylate formaldehyde, sodium hydrosulfite, isoascorbic acid, hydroxylamine sulfate and sodium bisulfite may be used at similar levels, optionally in combination with metal ions such as, for example iron and copper, optionally further including complexing agents for the metal. Chain transfer agents such as mercaptans may be used to lower the molecular weight of the polymer. The monomer mixture may be added neat or as an emulsion in water. The monomer mixture may be added in a single addition or more additions or continuously over the reaction period using a uniform or varying composition. Additional ingredients such as, for example, free radical initiators, oxidants, reducing agents, chain transfer agents, neutralizers, surfactants, and dispersants may be added prior to, during, or subsequent to the monomer addition. Processes yielding polymodal particle size distributions such as those disclosed in US Patent Nos. 4,384,056 and 4,539,361, for example, may be employed.

The emulsion polymer may be formed in a multi-stage emulsion polymerization process. In the multi-stage emulsion polymerization process at least two stages different in composition are formed in sequential fashion. Preferred is a two-stage emulsion polymerization process in which the weight of the first stage polymer is from 10% to 90%, preferably from 30% to 70%, of the total weight of the first stage polymer and the second stage polymer, based on dry polymer weights. The polymerization techniques used to prepare aqueous multi-stage emulsion-polymers are well known in the art such as, for example, as disclosed in U.S. Patents No. 4,325,856; 4,654,397; and 4,814,373.

A multi-stage emulsion polymerization process usually results in the formation of at least two mutually incompatible polymer compositions, thereby resulting in the formation of at least two phases. The mutual incompatibility of two polymer compositions and the resultant multiphase structure of the polymer particles may be determined in various ways known in the art. The use of scanning electron microscopy using staining techniques to emphasize the difference between the phases, for example, is such a technique. Such particles are composed of two or more phases of various geometries such as, for example, core/shell or core/sheath particles, core/shell particles with shell phases incompletely encapsulating the core, core/shell particles with a multiplicity of cores, and interpenetrating network particles. Each of the stages of the multi-staged emulsion polymer may contain the same monomers, surfactants, initiation system, chain transfer agents, etc. as disclosed herein-above for the emulsion polymer. In the case of a multi-staged polymer particle the physical characteristics of the emulsion polymer such as for example, acid monomer content, Tg, etc. for the purpose of this invention is to be calculated using the overall composition of the emulsion polymer without regard for the number of stages or phases therein. The emulsion polymer is also contemplated to be formed in two or more stages, the stages differing in molecular weight. Blending two different emulsion polymers is also contemplated.

The emulsion polymer can also be formed in a living radical polymerization LRP process. Suitable LRP processes include RAFT, ATRP, SET-LRP, and Nitroxide. Monomer, macromonomer, seed particles, and surface initiated polymerization; and initiators may be used. Polymer construction can be any of the forms currently known in the art, including random, block, multiblock, and gradient.

The average particle diameter of the emulsion polymer particles is typically from 40 nanometers to 1000 nanometers, preferably from 40 nanometers to 300 nanometers. Particle sizes herein are those measured by dynamic light scattering on a Brookhaven BI-90 analyzer.

The aqueous coating composition is prepared by techniques which are well known in the coatings art. First, pigment(s), if any, are well dispersed in an aqueous medium under high shear such as is afforded by a COWLES (R) mixer or predispersed colorant(s), or mixtures thereof are used. Then the emulsion polymer is added under low shear stirring along with the coalescent composition and other coatings adjuvants as desired. The aqueous coating composition may include, in addition to the emulsion polymer and optional pigment(s), film-forming or non-film-forming solution or other emulsion polymers in an amount of 0% to 200% by weight of the emulsion polymer, and conventional coatings

adjuvants such as, for example, extenders, emulsifiers, coalescing agents other than the coalescent composition of the present invention, plasticizers, antifreezes, curing agents, buffers, neutralizers, thickeners, rheology modifiers, humectants, wetting agents, biocides, plasticizers, antifoaming agents, UV absorbers, fluorescent brighteners, light or heat stabilizers, biocides, chelating agents, dispersants, colorants, waxes, and water-repellants.

Examples of suitable pigments and extenders include titanium dioxide such as anatase and rutile titanium dioxides; zinc oxide; antimony oxide; iron oxide; magnesium silicate; calcium carbonate; organic and inorganic colored pigments; aluminosilcates; silica; various clays such as kaolin and delaminated clay; and lead oxide. It is also contemplated that the aqueous coating composition may also contain opaque polymer particles, such as, for example, Ropaque™ Opaque Polymers (Dow Chemical Co.). Also contemplated are encapsulated or partially encapsulated opacifying pigment particles; and polymers or polymer emulsions adsorbing or bonding to the surface of pigments such as titanium dioxide; and hollow pigments, including pigments having one or more voids.

The amounts of pigment and extender in the aqueous coating composition vary from a pigment volume concentration (PVC) of 0 to 85 and thereby encompass coatings otherwise described in the art, for example, as clear coatings, stains, flat coatings, satin coatings, semi-gloss coatings, gloss coatings, primers, textured coatings, and the like. The pigment volume concentration is calculated by the following formula:

$$\text{PVC (\%)} = \frac{\text{volume of pigment(s), + volume extender(s) x 100.}}{\text{total dry volume of paint}}$$

The solids content of the aqueous coating composition may be from 10% to 70% by volume. The viscosity of the aqueous coating composition may be from 50 centipoises to 50,000 centipoises, as measured using a Brookfield viscometer; viscosities appropriate for different application methods vary considerably.

The aqueous coating composition herein expressly includes architectural, maintenance, and industrial coatings, caulks, sealants, and adhesives. The aqueous coating composition is typically applied to a substrate such as, for example, wood, metal, plastics, marine and civil engineering substrates, cementitious substrates such as, for example, concrete, stucco, and mortar, previously painted or primed surfaces, and weathered surfaces. The aqueous coating composition may be applied to a substrate using conventional coatings application methods such as, for example, brush, roller, caulking applicator, roll coating, gravure roll, curtain coater and spraying methods such as, for example, air-atomized spray, air-assisted spray, airless spray, high volume low pressure spray, and air-assisted airless spray.

Drying of the aqueous coating composition to provide a coating may be allowed to proceed under ambient conditions such as, for example, at 5 °C to 35 °C. or the coating may be dried at elevated temperatures such as, for example, from 35 °C to 150 °C.

**Experimental Methods**

MFFT Determination. MFFT Bar SS-3000 (Paul N. Gardener Co. Inc. 316 Northeast First Street, Pompano Beach, Florida, 33060) was used in the method based on ASTM D-2354.
Scrub: Scrub was determined based on ASTM D 2486-74A using a Byk-Gardener Abrasion Tester.
Block Resistance: Block was determined based on ASTM D 4946-89.
Flow/Leveling: Flow/leveling was determined using a Leneta Leveling Test Blade LTB-2 (The Leneta Company, 15 Whitney Road, Mahwah NJ 07430) based on ASTM D 4062-88.
Gloss: Gloss was determined using a Byk-Gardener Haze-Gloss Meter Model No 4601. (Byk-Gardener USA, Columbia, MD 21046)based on ASTM D 523-89.
Heat aged ("HA") conditions: 60°C oven: VWR Scientific 1380 FM forced air oven
Color measurements: Made using a XRite 8400; software X-RiteColor Master version 5.1.1; sample preparation was the same as used for gloss.

The following examples serve to illustrate the invention.

COMPARATIVE EXAMPLE 1. Synthesis of 3-(2-ethylhexyloxy)-1,2-propanediol.

2-Ethylhexanal (12.8 g, 15.6 ml, 0.1 mol), glycerol (92.09 g, 73.7 ml, 1 mol), and 10% Pd/C (5 wt %, 0.64 g) were charged to the Parr reactor, purged with nitrogen three times, heated to 200 °C with stirring and run at 1000 psi of hydrogen for 22 h. GC analysis showed complete consumption of 2-ethylhexanal. The reaction mixture was filtered, diluted with methanol and analyzed by GC. The system formed one phase in the presence of methanol (50 ml). The mixture contained glycerol monoethers 3-(2-ethyl)hexoxy-1,2-propanediol and 2-(2-ethyl)hexoxy-1,2-propanediol (73.8%, ratio 9.1), glycerol diethers 1,3-di(2-ethy)hexoxy-2-propanol and 2,3-di(2-ethyl)hexoxy-1-propanol (13.8), and

2-ethyhexanol (6.5%). Methanol was evaporated to form a two phase system, water (50 ml) was added, and the mixture was extracted with ether (50 ml x 4) and dried over sodium sulfate. The solvent was evaporated in vacuum to give the crude product (15.5 g), which was purified by column chromatography on silica gel with hexane-ethyl acetate from 10:1 to 4:1 to give 8.66 g of a mixture of 3-(2-ethylhexyloxy)-1,2-propanediol (major) and 2-(2-ethylhexyloxy)-1,3-propanediol (minor). The product was characterized by $^1$H and $^{13}$C NMR spectra.

COMPARATIVE EXAMPLE B Synthesis of 3-(3-phenylpropoxy)-1,2-propanediol.

*trans*-Cinnamaldehyde (13.2 g, 12.6 ml, 0.1 mol) of, glycerol (92.09 g, 73.7 ml, 1 mol) and 10% Pd/C in the Parr reactor (0.66 g, 5 wt% relative to the aldehyde) were charged to the Parr reactor, purged with nitrogen three times, heated to 200 °C with stirring and run at 1000 psi of hydrogen for 20 h. GC analysis showed complete consumption of the aldehyde. The mixture was filtered, the product was extracted by ether (50 ml x 5), and the combined ether solution was dried with sodium sulfate. The solvent was evaporated and the residue was chromatographed on silica gel using hexane-ethyl acetate from 5:1 to 1:1 to give 4.6 g of the monoether (95% purity). This product was approximately a 2:1 mixture of 3-(3-phenylpropyl)-1,2-propanediol and 3-(3-cyclohexylpropyl)-1,2-propanediol. The major component of this mixture was separated by second column chromatography and characterized by $^1$H and $^{13}$C NMR.

COMPARATIVE EXAMPLE C. Synthesis 1,3-bis(decyloxy)-2-propanol.

n-Decanal (15.6 g, 18.8 ml, 0.1 mol), glycerol (92.09 g, 73.7 ml, 1 mol), and 10% Pd/C (0.78 g, 5 wt % relative to the aldehyde) were charged to the Parr reactor, purged with nitrogen three times, heated to 200 °C with stirring and run at 1000 psi of hydrogen for 22 h. GC analysis showed complete consumption of n-decanal. The reaction mixture was filtered, diluted with methanol (50 ml) and analyzed by GC. The system formed one phase in the presence of methanol. The mixture contained glycerol monoethers glycerol monoethers 3-decyloxy-1,2-propanediol and 2-decyloxy-1,2-propanediol (60.6, ratio 6.9), glycerol diethers 1,3-didecyloxy-2-propanol and 2,3-didecyloxy-1-propanol (18.9%), and n-decanol (4.9%). Methanol was evaporated to form a two phase system, water (50 ml) was added, and the mixture was extracted with ether (50 ml x 4) and dried over sodium sulfate. The solvent was evaporated in vacuum to give the crude product (16.9 g), which was purified by column chromatography on silica gel with hexane-ethyl acetate from 10:1 to 3:1 to give 8.45 g of 3-decyloxy-1,2-propanediol (major) and 2-decyloxy-1,3-propanediol (minor). The product was characterized by $^1$H and $^{13}$C NMR spectra. Pure 3-decyloxy-1,2-propanediol was isolated from chromatography fraction 25, melting point 38.5-39 °C.

EXAMPLE 1. Formation of 1,3-bis(decyloxy)-2-propanol

Step 1. Preparation of 3-decyloxy-1,2-propanediol

n-Decanal (46.88 g, 56.34 ml, 0.30 mol), glycerol (165.76 g, 131.36 ml, 1.80 mol), and 5% Pd/C (2.34 g, 5 wt % relative to the aldehyde) were charged to the Parr reactor, purged with hydrogen (100 psi) three times, then charged 500 psi of hydrogen, heated to 200 °C with stirring and ran at 1000 psi of hydrogen for 8 h. GC analysis showed complete consumption of n-decanal. The reaction mixture was filtered, the top (product) phase was separated (65.33 g) and the bottom (glycerol) phase was diluted with water (1:1) and extracted with diethyl ether, 100 mL x 3. The combined ether extracts were dried with sodium sulfate. The solvent was evaporated to give the material (4.75 g), which was combined with the recovered earlier top (product) phase to give 70.08 g of the crude 3-decyloxy-1,2-propanediol.

Step 2. Preparation of 1,3-bis(decyloxy)-2-propanol

n-Decanal (9.38 g, 11.27 ml, 0.06 mol), crude 3-decyloxy-1,2-propanediol prepared above without purification (68.32 g, ~0.3 mol), and 5%Pd/C catalyst (0.47 g, 5wt% relative to the aldehyde) were charged to the Parr reactor, purged with hydrogen three times, heated to 200°C, and run at 1000 psi for about 6 h. Then additional n-decanal (9.38 g, 11.27 ml, 0.06 mol) was introduced and the reaction was carried out at 210°C and 1000 psi of hydrogen for 22 h. Analysis of the mixture revealed about 30.5 GC area % of 1,3-bis(decyloxy)-2-propanol. A vacuum distillation of the crude product resulted in 26.1 g of 1,3-bis(decyloxy)-2-propanol, boiling point 159-160 °C/0.03 mm Hg. The product was characterized by $^1$H and $^{13}$C NMR spectra. EXAMPLE 2. Formation of 1,1'-oxybis[3-(heptyloxy)]-2-propanol (new composition of matter)

Step 1. Preparation of 3-[2-hydroxy-3-(heptyloxy)propoxy]-1,2-propanediol

Commercial diglycerol from TCI America (199.40 g, 155.78 ml, 1.2 mol), heptanal (27.4 g, 33.5 ml, 0.24 mol),

and 5%Pd/C, 1.37 g 5 wt% relative to the aldehyde) were charged to the Parr reactor, purged with hydrogen three times. Then the reaction mixture was heated to 200 °C with stirring and run at 1000 psi for 18 hrs. The mixture was filtered off from the reactor, and the reaction was carried out again using new starting materials in the same amounts and the old catalyst remaining in the reactor. The second reaction mixture was combined with the first one, water (200 mL) was added, and the solution was extracted with diethyl ether 200 mL x 10. The combined ether phases were dried with sodium sulfate, ether was evaporated to give the crude monoether, 3-[2-hydroxy-3-(heptyloxy)propoxy]-1,2-propanediol. Step 2. Preparation of 1,1'-oxybis[3-(heptyloxy)]-2-propanol A portion of the above material (100.5 g, 0.380 mol), heptanal (13.7 g, 16.8 ml, 0.12 mol), and (0.69 g of 5%Pd/C, 5 wt% relative to the aldehyde) was charged into the Parr reactor, purged with hydrogen three times, heated to 200 °C with stirring and run at 1000 psi for 8 hrs. Then additional heptanal (13.7 g, 16.8 ml, 0.12 mol) was charged to the current reaction mixture and purged with hydrogen three times. The mixture was heated to 200 °C and run at 1000 psi for 6 hrs. The crude product was fractionally distilled in vacuum to give 79.3 g of 3-[2-hydroxy-3-(heptyloxy)propoxy]-1,2-propanediol, boiling point 164-165 °C/0.05 mm Hg, and 17.85 g of 1,1'-oxybis[3-(heptyloxy)]-2-propanol as the residue. The product was characterized by $^{1}H$ and $^{13}C$ NMR spectra.

EXAMPLE 3. Evaluation of MFFT

Coalescent samples were evaluated for minimum film formation temperature ("MFFT") in the following coating composition.

Table 3.1. Coating composition

| **Grind** | | pounds | gallons |
|---|---|---|---|
| | Water | 170.27 | 20.40 |
| | OROTAN™ 731A | 0.70 | 0.08 |
| | TERGITOL™ 15-S-15 | 2.00 | 0.23 |
| | KATHON™ LXE | 1.25 | 0.15 |
| | KRONOS™ 4311 | 261.58 | 13.13 |
| | RHOPLEX™ VSR-1050 | 419.85 | 47.98 |
| | ROPAQUE™ Ultra | 49.56 | 5.80 |
| | Propylene Glycol | 12.52 | 1.45 |
| | *Grind Sub-total* | 917.74 | 89.22 |
| **LetDown** | | 917.74 | |
| | BYK-024 | 1.42 | 0.17 |
| | AMP-95 | 0.50 | 0.06 |
| | Water | 29.00 | 8.87 |
| | ACRYSOL™ RM-8W | 6.00 | 0.68 |
| | ACRYSOL™ RM-2020 | 50.00 | 0.57 |
| | coalescent | 3.99 | |
| | Totals | 1008.65 | 100.07 |

The aqueous coating composition was measured out to 60.0 g and 0.30g of coalescent (or water in the case of the "none" control) was added and mixed in a DAC 150 FVZ FlackTek Speedmixer. Note that several of the test coalescents required warming to effect incorporation.

Table 3.2 MFFT determination and results

| Coalescent | MFFT °C | HA MFFT |
|---|---|---|
| NONE | 12.5 | 11.5 |
| TEXANOL™ | 10.5 | 10.87 |
| OPTIFILM™ 400 | 9.04 | 8.96 |
| 3-(3-phenyl-propoxy)-1,2-propanediol | 10.88 | 11.9 |
| 3-(2-ethylhexyloxy)-1,2-propanediol | 10.5 | 10.24 |
| 1,3-bis(decyloxy)-2-propanol | 9.04 | 7.64 |
| 3-decyloxy-1,2-propanediol | 11.92 | 9.18 |

(continued)

| | MFFT | HA |
|---|---|---|
| 1,1'-oxybis[3-(heptyloxy)]-2-propanol | 9.08 | 9.4 |

Coalescent Compositions Example 1(1,3-bis(decyloxy)-2-propanol) and Example 2(1,1'-oxybis[3-(heptyloxy)]-2-propanol) of this invention are seen to have desirably effective MFFT performance( lower MFFT is better).

EXAMPLE 4.Scrub resistance evaluation and results

Table 4.1 Scrub resistance results

| | left | | right | | average | |
|---|---|---|---|---|---|---|
| Coalescent | L first cut | L full cut | R first cut | R full cut | Av first cut | Av full cut |
| NONE | 932 | 1831 | 1260 | 2020 | 1096 | 1925.5 |
| TEXANOL™ | 1182 | 1869 | 1405 | 2435 | 1293.5 | 2152 |
| OPTIFILM™ 400 | 1185 | 2075 | 1560 | 2598 | 1372.5 | 2336.5 |
| 3-(3-phenyl-propoxy)-1,2-propanediol | 1085 | 1745 | 1341 | 1976 | 1213 | 1860.5 |
| 3-(2-ethylhexyloxy)-1,2-propanediol | 1182 | 1788 | 1521 | 2150 | 1351.5 | 1969 |
| 1,3-bis(decyloxy)-2-propanol | 1310 | 2210 | 1587 | 2600 | 1448.5 | 2405 |
| 3-decyloxy-1,2-propanediol | 1235 | 1955 | 1703 | 2635 | 1469 | 2295 |
| 1,1'-oxybis[3-(heptyloxy)]-2- | 1207 | 2212 | 1152 | 2433 | 1179.5 | 2322.5 |

Coalescent Compositions Example 1(1,3-bis(decyloxy)-2-propanol) and Example 2(1,1'-oxybis[3-(heptyloxy)]-2-propanol) of this invention are seen to have desirably effective scrub resistance performance (larger scrub numbers are better).

EXAMPLE 5. Block resistance evaluation and results

Block resistance testing was carried out using a 3mil applicator, 50°C oven for 30 minutes, 1000g+stopper (127 g/cm2 (1.8 psi))]. Block resistance was rated on a 10 point scale and larger numbers are better.

Table 5.1 Block resistance results

| | Block 6hr | Block 24hr | Block 7day |
|---|---|---|---|
| NONE | 7.5 | 8.5 | 8.5 |
| TEXANOL™ | 7 | 8 | 7 |
| OPTIFILM™ 400 | 7 | 7 | 7 |
| 3-(3-phenyl-propoxy)-1,2-propanediol | 6 | 7 | 6 |
| 3-(2-ethylhexyloxy)-1,2-propanediol | 3 | 6 | 5 |
| 1,3-bis(decyloxy)-2-propanol | 6 | 6 | 6 |
| 3-decyloxy-1,2-propanediol | 0 | 5 | 4 |
| 1,1'-oxybis[3-(heptyloxy)]-2-propanol | 6 | 6 | 6 |

Table 5.2 Repeated block resistance tests

| | Block 6hr | Block 24hr |
|---|---|---|
| NONE | 5 | 8 |
| OPTIFILM™ 400 | 4 | 7 |
| 1,3-bis(decyloxy)-2-propanol Trial 1 | 5 | 7 |
| 1,3-bis(decyloxy)-2-propanol Trial 2 | 5 | 7 |

(continued)

| | Block 6hr | Block 24hr |
|---|---|---|
| 1st 1,1'-oxybis[3-(heptyloxy)]-2-propanol Trial 1 | 4 | 6 |
| 2nd 1,1'-oxybis[3-(heptyloxy)]-2-propanol Trial 2 | 5 | 7 |

Coalescent Compositions Example 1(1,3-bis(decyloxy)-2-propanol) and Example 2(1,1'-oxybis[3-(heptyloxy)]-2-propanol) of this invention are seen to have desirably effective block resistance performance (higher block numbers are better).

EXAMPLE 6. Flow and Leveling F/L evaluation and results

Table 6.1 Flow and leveling results

| Coalescent | F/L |
|---|---|
| NONE | 10 |
| TEXANOL™ | 10 |
| OPTIFILM™ 400 | 10 |
| 3-(3-phenyl-propoxy)-1,2-propanediol | 10 |
| 3-(2-ethylhexyloxy)-1,2-propanediol | 10 |
| 1,3-bis(decyloxy)-2-propanol | 10 |
| 3-decyloxy-1,2-propanediol | 10 |
| 1,1'-oxybis[3-(heptyloxy)]-2-propanol | 10 |

Coalescent Compositions Example 1(1,3-bis(decyloxy)-2-propanol) and Example 2(1,1'-oxybis[3-(heptyloxy)]-2-propanol) of this invention are seen to have desirably effective flow and leveling performance (10 point scale; higher numbers are better).

EXAMPLE 7. Gloss determination and results

Table 7.1. Gloss results

| | 60 degree |
|---|---|
| NONE | 73.9 |
| TEXANOL™ | 74.3 |
| OPTIFILM™ 400 | 75.9 |
| 3-(3-phenyl-propoxy)-1,2-propanediol | 79.6 |
| 3-(2-ethylhexyloxy)-1,2-propanediol | 78.9 |
| 1,3-bis(decyloxy)-2-propanol | 77.4 |
| 3-decyloxy-1,2-propanediol | 80.8 |
| 1,1'-oxybis[3-(heptyloxy)]-2-propanol | 80.0 |

Table 7.2. Gloss results

| | 20 degree |
|---|---|
| NONE | 36.8 |
| TEXANOL™ | 40.4 |
| OPTIFILM™ 400 | 40.9 |
| 3-(3-phenyl-propoxy)-1,2-propanediol | 35.3 |
| 3-(2-ethylhexyloxy)-1,2-propanediol | 30.1 |
| 1,3-bis(decyloxy)-2-propanol | 41.0 |

(continued)

| | 20 degree |
|---|---|
| 3-decyloxy-1,2-propanediol | 50.3 |
| 1,1'-oxybis[3-(heptyloxy)]-2-propanol | 39.7 |

Coalescent Compositions Example 1(1,3-bis(decyloxy)-2-propanol) and Example 2(1,1'-oxybis[3-(heptyloxy)]-2-propanol) of this invention are seen to have desirably high 20° and 60° gloss(larger is better).

EXAMPLE 8. L*a*b* and DE results

Table 8.1. L*a*b* and DE results

| Coalescent | DL | da | db | DE | Lb/Lw |
|---|---|---|---|---|---|
| NONE | -0.89 | -0.27 | -0.87 | 1.274 | 99.087 |
| TEXANOL™ | 0.94 | -0.3 | 0.99 | 1.398 | 99.035 |
| OPTIFILM™ 400 | -1.02 | -0.31 | -1.05 | 1.496 | 98.953 |
| 3-(3-phenyl-propoxy)-1,2-propanediol | -1.25 | -0.39 | -1.30 | 1.845 | 98.716 |
| - 3-(2-ethylhexyloxy)-1,2-propanediol | 0.94 | -0.35 | -1.10 | 1.489 | 99.035 |
| 1,3-bis(decyloxy)-2-propanol | -1.07 | -0.35 | -1.17 | 1.624 | 98.902 |
| 3-decyloxy-1,2-propanediol | -0.96 | -0.35 | -1.05 | 1.465 | 99.015 |
| 1,1'-oxybis[3-(heptyloxy)]-2-propanol | -1.11 | -0.32 | -1.04 | 1.550 | 98.860 |

Coalescent Compositions Example 1(1,3-bis(decyloxy)-2-propanol) and Example 2(1,1'-oxybis[3-(heptyloxy)]-2-propanol) of this invention are seen to have acceptable opacity (DL*) and delta a* values (smaller is better).

Coalescent Compositions Example 1(1,3-bis(decyloxy)-2-propanol) and Example 2(1,1'-oxybis[3-(heptyloxy)]-2-propanol) of this invention are seen to have acceptable delta b* and DE values (smaller is better).

EXAMPLE 9 Heat aged L*, a*, b* evaluation and results.

Initial color values L*, a*, b* (3 mil were measured over black Leneta 5C charts with an X-Rite Color Master 8400 Colorimeter)

Table 9.1 Initial color values

| | L* | a* | b* |
|---|---|---|---|
| NONE | 96.56 | -1.10 | 0.08 |
| TEXANOL™ | 96.44 | -1.13 | 0.08 |
| OPTIFILM™ 400 | 96.41 | -1.15 | 0.04 |
| 3-(3-phenyl-propoxy)-1,2-propanediol | 96.09 | -1.18 | -0.14 |
| 3-(2-ethylhexyloxy)-1,2-propanediol | 96.51 | -1.16 | 0.13 |
| 1,3-bis(decyloxy)-2-propanol | 96.42 | -1.15 | 0.02 |
| 3-decyloxy-1,2-propanediol | 96.55 | -1.15 | 0.12 |
| 1,1'-oxybis[3-(heptyloxy)]-2-propanol | 96.30 | -1.17 | 0.00 |

Table 9.2 Heat Aged color values

| Coalescent | L* | a* | b* |
|---|---|---|---|
| NONE | 97.32 | -1.20 | 1.22 |
| TEXANOL™ | 96.39 | -1.24 | 0.35 |
| OPTIFILM™ 400 | 96.70 | -1.22 | 0.65 |
| 3-(3-phenyl-propoxy)-1,2-propanediol | 96.53 | -1.22 | 0.55 |
| 3-(2-ethylhexyloxy)-1,2-propanediol | 96.41 | -1.24 | 0.45 |
| 1,3-bis(decyloxy)-2-propanol | 96.79 | -1.21 | 0.71 |
| 3-decyloxy-1,2-propanediol | 96.52 | -1.23 | 0.50 |
| 1,1'-oxybis[3-(heptyloxy)]-2-propanol | 96.46 | - | 0.44 |

Ideally, L* values should not change much on heat aging. Coalescent Compositions Example 1(1,3-bis(decyloxy)-2-propanol) and Example 2(1,1'-oxybis[3-(heptyloxy)]-2-propanol) of this invention are seen to have acceptable L (larger is better) and heat aged L values (equal to initial is better, and greater-than is better than less-than initial); a* and b* values (smaller is better, and smaller change is better) initially and after heat aging are acceptable.

EXAMPLES 10-14. Preparation of various coalescents

Examples 10-14 of the present invention were prepared according to the teachings of Examples 1- 2. The coalescent compositions were:

Example 10. 1,3-bis(2-phenylethoxy)-2-propanol

Example 11. 1,3-bis(2-ethylhexyloxy)-2-propanol

Example 12. 1,3-bis(3,5,5-trimethylhexyloxy)-2-propanol

Example 13. 1,3-bis(3,7-dimethyloctyloxy)-2-propanol

Example 14. 1,1'-oxybis[3-(octyloxy)]-2-propanol (sample 6)

EXAMPLE 15. Evaluation of Coalescents of Examples 10-14.

Evaluations were carried out as in Examples 3-9. Results follow.

Table 18.1 Evaluation of coalescents of Examples 10-14.

| Coalescent | MFFT, C | Block 6hr dry time | Block 24hr dry time | Gloss 20 | Gloss 60 | Gloss 20 7d | Gloss 60 7d | scrub resistance |
|---|---|---|---|---|---|---|---|---|
| NONE | 12.5 | 6 | 8 | 38.3 | 72.4 | 26.4 | 71.6 | 496 |
| OPTIFILM™ 400 | 7.8 | 5 | 6 | 45 | 75.8 | 36.6 | 77 | 610 |
| 1,3-bis(decyloxy)-2-propanol | 8.2 | 5 | 4 | 19.3 | 55.4 | 9.2 | 41.1 | 599 |
| 1,1'-oxybis [3-(heptyloxy)]-2-propanol | 10.4 | 0 | 4 | 50.4 | 77.7 | 36 | 79.1 | 554 |
| OPTIFILM™ 300 | 7.4 | 4 | 6 | 38.1 | 72.1 | 29.9 | 73.6 | 544 |

(continued)

| Coalescent | MFFT, C | Block 6hr dry time | Block 24hr dry time | Gloss 20 | Gloss 60 | Gloss 20 7d | Gloss 60 7d | scrub resistance |
|---|---|---|---|---|---|---|---|---|
| 1,3-bis(2-phenylethoxy)-2-propanol (example 10) | 8.1 | 6 | 7 | 45.9 | 76.1 | 39 | 78.8 | 574 |
| 1,3-bis(2-ethylhexyloxy)-2-propanol (example 11) | 5 | 6 | 6 | 45.2 | 75.4 | 38 | 79.6 | 580 |
| 1,3-bis(3,5,5-trimethylhexyloxy)-2-propanol (example 12) | 7.7 | 5 | 5 | 45.6 | 76 | 35.1 | 77.7 | 509 |
| 1,3-bis(3,7-dimethyloctyloxy)-2-propanol (example 13) | 8.6 | 5 | 7 | 40.5 | 71.8 | 15.4 | 54.7 | 556 |
| 1,1'-oxybis [3-(octyloxy)]-2-propanol (example 14) | 10.5 | 3 | 4 | 51.7 | 78 | 40.1 | 81.2 | 445 |

Table 18.2. Evaluation of coalescents of Examples 13-17.

| Coalescent | L*/w | a*/w | b*/w | C*/w | h°/w | L*/b | a*/b | b*/b | dL* | da* | db* |
|---|---|---|---|---|---|---|---|---|---|---|---|
| NONE | 97.43 | -0.85 | 1.18 | 1.45 | 125.88 | 96.19 | -1.16 | -0.04 | 0.987 | -0.3 | -1.2 |
| OPTIFILM™ 400 | 97.6 | -0.85 | 1.22 | 1.48 | 125.01 | 96.53 | -1.12 | 0.16 | 0.989 | -0.3 | -1.1 |
| 1,3-bis(decyloxy)-2-propanol | 97.52 | -0.85 | 1.22 | 1.48 | 124.82 | 96.51 | -1.13 | 0.21 | 0.99 | -0.3 | -1 |
| 1,1'-oxybis [3-(heptyloxy)]-2-propanol | 97.46 | -0.85 | 1.21 | 1.48 | 125.07 | 96.34 | -1.14 | 0.1 | 0.989 | -0.3 | -1.1 |
| OPTIFILM™ 300 | 97.53 | -0.86 | 1.25 | 1.51 | 124.41 | 96.71 | -1.12 | 0.3 | 0.992 | -0.3 | -1 |
| 1,3-bis(2-phenylethoxy)-2-propanol (example 10) | 97.59 | -0.86 | 1.24 | 1.51 | 124.86 | 96.59 | -1.12 | 0.25 | 0.99 | -0.3 | -1 |
| 1,3-bis(2-ethylhexyloxy)-2-propanol (example 11) | 97.63 | -0.85 | 1.25 | 1.52 | 124.28 | 96.65 | -1.11 | 0.25 | 0.99 | -0.3 | -1 |
| 1,3-bis(3,5,5-trimethylhexyloxy)-2-propanol (example 12) | 97.65 | -0.85 | 1.25 | 1.51 | 124.27 | 96.65 | -1.12 | 0.25 | 0.99 | -0.3 | -1 |
| 1,3-bis(3,7-dimethyloctyloxy)-2-propanol (example 13) | 97.57 | -0.85 | 1.21 | 1.48 | 125.19 | 96.5 | -1.12 | 0.13 | 0.989 | -0.3 | -1.1 |

(continued)

| Coalescent | L*/w | a*/w | b*/w | C*/w | h°/w | L*/b | a*/b | b*/b | dL* | da* | db* |
|---|---|---|---|---|---|---|---|---|---|---|---|
| 1,1'-oxybis [3-(octyloxy)]-2-propanol (example 14) | 97.62 | -0.83 | 1.28 | 1.53 | 123.08 | 96.64 | -1.12 | 0.24 | 0.99 | -0.3 | -1 |

Coalescent Compositions of Examples 10-14 of this invention are effective coalescents and form coatings with useful levels of performance.

## Claims

1. A coalescent composition selected from the group consisting of: 1,3-($C_6$-$C_{12}$alkyloxy)-2-propanol; 1,1'-oxybis [3-($C_6$-$C_{12}$alkyloxy)]-2-propanol; and mixtures and isomers thereof.

2. The coalescent composition of claim 1 selected from the group consisting of: 1,3-(decyloxy)-2-propanol and 1,1'-oxybis[3-(heptyloxy)]-2-propanol.

3. A method for forming glycerol diethers or diglycerol diethers comprising:

   1) subjecting an aldehyde and a molar excess of glycerol or diglycerol to reductive etherification conditions to form a glycerol monoether or a diglycerol monoether; and
   2) subjecting an aldehyde and a molar excess of said glycerol monoether or said diglycerol monoether to reductive etherification conditions to form said glycerol diethers or said diglycerol diethers.

4. The method of claim 3 wherein said glycerol diethers or diglycerol diethers are selected from the group consisting of: 1,3-($C_6$-$C_{12}$alkyloxy)-2-propanol; 1,1'-oxybis[3-($C_6$-$C_{12}$alkyloxy)]-2-propanol; and mixtures and isomers thereof.

5. An aqueous coating composition comprising an emulsion polymer and from 0.1% to 40% by weight, based on the weight of emulsion polymer solids, said coalescent composition of claim 1 or claim 2.

6. The aqueous coating composition of claim 3 wherein said emulsion polymer has a MFFT of from -5 °C to 100 °C, said coating composition comprising from 0.1% to 30% by weight, based on the weight of emulsion polymer solids, said coalescent composition of claim 1 or claim 2.

7. A method for forming a coating comprising

   (a) forming said aqueous coating composition of claim 5 or claim 6;
   (b) applying said aqueous coating composition to a substrate; and
   (c) drying, or allowing to dry, said applied aqueous coating composition.

Europäisches Patentamt
European Patent Office
Office européen des brevets

# EUROPEAN SEARCH REPORT

Application Number
EP 11 17 5610

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GB 1 198 387 A (AGFA GEVAERT AG [DE]) 15 July 1970 (1970-07-15) * table * | 1,2 | INV. C07C41/09 C07C43/10 C09D5/02 |
| X | US 2002/004605 A1 (LEE BYUNG MIN [KR] ET AL) 10 January 2002 (2002-01-10) * table 2 * | 1,2 | |
| X | WO 2008/134387 A1 (DOW GLOBAL TECHNOLOGIES INC [US]; THOEN JOHAN A [BE]; BARTELINK CAMIEL) 6 November 2008 (2008-11-06) * example 1 * | 1 | |
| A | US 2010/048940 A1 (TULCHINSKY MICHAEL L [US] ET AL) 25 February 2010 (2010-02-25) * example 21 * | 1-7 | |

TECHNICAL FIELDS SEARCHED (IPC)

C07C
C09D

The present search report has been drawn up for all claims

1

| Place of search | Date of completion of the search | Examiner |
|---|---|---|
| The Hague | 30 January 2012 | O'Sullivan, Paul |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 11 17 5610

This annex lists the patent family members relating to the patent documents cited in the above-mentioned European search report.
The members are as contained in the European Patent Office EDP file on
The European Patent Office is in no way liable for these particulars which are merely given for the purpose of information.

30-01-2012

| Patent document cited in search report | | Publication date | Patent family member(s) | | | Publication date |
|---|---|---|---|---|---|---|
| GB 1198387 | A | 15-07-1970 | FR | 1599022 | A | 15-07-1970 |
| | | | GB | 1198387 | A | 15-07-1970 |
| US 2002004605 | A1 | 10-01-2002 | JP | 2002003428 | A | 09-01-2002 |
| | | | KR | 20020001905 | A | 09-01-2002 |
| | | | US | 2002004605 | A1 | 10-01-2002 |
| WO 2008134387 | A1 | 06-11-2008 | CA | 2685315 | A1 | 26-10-2009 |
| | | | CN | 101668727 | A | 10-03-2010 |
| | | | EP | 2152652 | A1 | 17-02-2010 |
| | | | JP | 2010525074 | A | 22-07-2010 |
| | | | RU | 2009143877 | A | 10-06-2011 |
| | | | US | 2010179354 | A1 | 15-07-2010 |
| | | | WO | 2008134387 | A1 | 06-11-2008 |
| US 2010048940 | A1 | 25-02-2010 | CN | 102131757 | A | 20-07-2011 |
| | | | EP | 2318346 | A1 | 11-05-2011 |
| | | | US | 2010048940 | A1 | 25-02-2010 |
| | | | WO | 2010027663 | A1 | 11-03-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20100048940 A
- US 5446210 A
- US 6011071 A
- US 4384056 A
- US 4539361 A
- US 4325856 A
- US 4654397 A
- US 4814373 A


### Non-patent literature cited in the description


- **A. TOUSSAINT ; M. DE WILDE ; F. MOLENAAR ; J. MULVIHILL.** Calculation of Tg and MFFT depression due to added coalescing agents. *Progress in Organic Coatings,* 1997, vol. 30, 179-184